# EUROPEAN PATENT SPECIFICATION

(11) **EP 1 221 966 B1**
(45) Date of publication and mention of the grant of the patent: **16.11.2005**
(21) Application number: 00961714.3
(22) Date of filing: 11.09.2000
(51) Int. Cl.: A61K 38/29, A61P 35/00

(54) **USE OF A PARATHYROID HORMONE FOR REDUCING THE RISK OF CANCER**
VERWENDUNG EINER PARATHYROIDHORMONE ZUR REDUKTION DES KREBSRISIKOS
UTILISATION D'UNE HORMONE PARATHYROIDE POUR REDUIRE LES RISQUES DE CANCER

(30) Priority: 20.09.1999 US 155455 P
(43) Date of publication of application: 17.07.2002
(73) Proprietor: ELI LILLY AND COMPANY, Indianapolis, Indiana 46285 (US)
(72) Inventor: HOCK, Janet, M., Indianapolis, IN 46250 (US)
(74) Representative: Kent, Lindsey Ruth
(86) International application number: PCT/US2000/024746
(87) International publication number: WO 2001/021198

(56) References cited:
- EP-A- 0 197 514
- EP-A- 0 878 201
- WO-A-96/03437
- US-A- 5 840 690
- TREMBLING P ET AL: "Comparison of effects of PTHrP 1-34, PTHrP 1-86 and PTH on proliferation of breast cancer cells." JOURNAL OF ENDOCRINOLOGY, vol. 144, no. SUPPL., 1995, page P223 XP000982364 ISSN: 0022-0795
- DELMAS P D ET AL: "Bone loss induced by cancer treatment and its management." EUROPEAN JOURNAL OF CANCER, vol. 34, no. 2, February 1998 (1998-02), pages 260-262, XP000982362 ISSN: 0959-8049
- AIGINGER P ET AL: "THERAPY OF MULTIPLE BONE METASTASES WITH PARATHYROID HORMONE AND RADIO PHOSPHORUS" OESTERREICHISCHE ZEITSCHRIFT FUER ONKOLOGIE, vol. 2, no. 1, 1975, pages 17-24, XP000982314 ISSN: 0377-2004

## Description

### TECHNICAL FIELD

This invention relates to the use of a parathyroid hormone to decrease the risk of developing carcinoma. More particularly, the invention relates to the use of a parathyroid hormone to decrease the risk of carcinoma in a person at risk of developing carcinoma, including persons at relatively low risk of osteoporosis, or at high risk of or suffering from osteoporosis. The invention relates more particularly to the use of a parathyroid hormone to decrease risks of breast or skin carcinoma.

### BACKGROUND OF THE INVENTION

Cancers are cellular tumors, or masses, that, when not treated, grow. Untreated cancers typically invade other tissues, spread, and are fatal.

Many human cancers are thought to involve genetic mutations. These genetic mutations result in, for example, the conversion of protooncogenes to oncogenes and/or dysfunction of tumor suppressor genes. Some of these mutations appear to be inherited or to cluster in families. Thus, a person with a family member that has had cancer may be at increased risk of cancer. Other genetic mutations occur spontaneously by, for example, exposure to a carcinogenic agent. Subjects carrying particular genetic mutations of particular genes, such as *the p53, BRCA1* and *RB1* genes, are at increased risk of developing certain types of cancer, including breast cancer and retinoblastoma.

Nonetheless, about 95% of breast cancer cases are thought to be sporadic, having a cause other than an inherited genetic mutation. Additional risk factors for cancers such as breast cancer include increasing age, exposure to a chemical carcinogen, to an immunosuppressive drug, or to a viral infection, and physical factors such as radiation. With these many risk factors, some estimate that 10% of the female human population will develop breast cancer during their lifetime. More particularly, for instance, populations considered to be at relatively high risk of breast cancer can be defined using the Gail model. Gail MH, Brinton LA, Byar DP et al (1989) Projecting individualized probabilities of developing breast cancer for white females who are being examined annually. J Natl Cancer Inst 81:1879-1886.

Hormones such as estrogen have been associated with the etiology of particular cancers such as breast and endometrial cancer. For instance, see Cauley JA Lucas FL, Kuller LH, Stone K, Browner W, Cummings SR (1999). Elevated serum estradiol and testosterone concentrations are associated with a high risk of breast cancer. Study of Osteoporotic Fractures Research Group. Annals of Inter Med. 130:270-277. Paradoxically, a significant portion of the population at risk for breast cancer is being treated with estrogen. Although subjects with osteoporosis and low bone mass are less likely to exhibit breast cancer than other women, avoiding estrogen therapy can still be desirable. Accordingly, forms of therapy, other than estrogen replacement therapy, for the treatment of osteoporosis and/or the reduction of risk of bone trauma are needed, and methods for reducing the risk of breast cancer that also treat or reduce the risk of osteoporosis would be beneficial.

Therefore, an evaluation of the ability of therapeutic compositions useful for treating osteoporosis to reduce the risk of breast cancer is a matter of pressing need. Demonstrations of ability of a composition to reduce the risk of breast cancer in human subjects are most convincing in clinical trials using such subjects, but this ability may also be usefully predicted using recognized animal models. See, for instance, Anzano MA, Peer CW, Smith JM et al (1996) Chemoprevention of mammary carcinogenesis in the rat: Combined use of raloxifene and 9-cis-retinoic acid. J Natl Cancer Inst 88:123-125.

Parathyroid hormone (PTH) is a therapeutic agent which has beneficial bone forming properties. Parathyroid hormone (PTH) is a secreted, 84 amino acid product of the mammalian parathyroid gland that controls serum calcium levels through its action on various tissues, including bone. The N-terminal 34 amino acids of bovine and human PTH (PTH(1-34)) is deemed biologically equivalent to the full length hormone. Other amino terminal fragments of PTH (including 1-31 and 1-38 for example), or PTHrP (PTH-related peptide/protein) or analogues of either or both, that activate the PTH/PTHrP receptor (PTH1 receptor) have shown similar biologic effects on bone mass, although the magnitude of such effects may vary.

The perceived roles of PTH and the PTH-related protein (PTHrP) in breast cancer remain unclear and have changed over time. PTH was once implicated as a possible agent in promoting the skeletal metastases associated with breast cancer. Subsequently, the PTHrP has been determined to be the causative agent of humoral hypercalcemia of malignancy, and a key factor in promoting skeletal metastases of solid tumors. The scientific literature reports that those relatively rare patients with both hyperparathyroidism, which increases PTH levels, and breast cancer exhibit significantly longer survival than breast cancer patients without hyperparathyroidism. Still, many studies focus on the role of PTHrP in breast cancer.

For example, increased serum PTHrP has been used as a diagnostic to search for an occult tumor. One study found that an increase in serum PTHrP had a 69% predictive value of death within 100 days. However, another study failed to find predictive value in PTHrP levels. Another study supports the assertion that, as a diagnostic, increased PTHrP does not predict additional breast cancer cases beyond those already identified by the current markers.

Other workers maintain that PTHrP levels may be predictive of the progression of breast cancer. For example, one study indicates that increased PTHrP is found in hypercalcemia of malignancy and when skeletal metastases are present. Other reports indicate that although there is a significant correlation between the expression of PTHrP and proliferation markers, this correlation does not necessarily exist between PTHrP and hypercalcemia. A retrospective study of breast cancer cells showed various promoter-initiated transcripts and PTHrP 1-139 mRNA in cases that progressed to metastases, especially bone metastases. Certain of the promoter-initiated transcripts were associated with the absence of estrogen receptors on the breast tumors.

However, the sample size of these studies was small and PTHrP may also be expressed in 30% of normal breast tissue. Further, there is also significant heterogeneity in the responsiveness of cell clones isolated from breast cancer. This heterogeneity was evidenced through expression of the PTH1 receptor, the mitogenic response, and the tumor cell's ability to invade Matrigel substrate when exposed to PTHrP fragments *in vitro.* Therefore, some workers conclude that PTHrP levels may not be predictive of even metastasis.

Studies of the receptors for PTH and PTHrP have not clarified the roles of these proteins in breast cancer. Immunocytochemical and *in situ* histohybridization studies show both PTHrP and PTH1 receptor (PTH1R) can be localized to malignant breast lesions. Between 50% and 70% of human breast cancers express PTHrP, and 50-96% express the PTH1R. Isolated breast cancer cells lines show PTHrP and/or the PTH1 receptor in both estrogen-positive (ER+) and estrogen-negative (ER-) cells. The ER+ breast cancer cell line, MCF-7, which produces PTHrP and expresses PTH1R proliferates in response to PTHrP in vitro. Although PTH and PTHrP bind competitively to the PTH1 receptor, there is no evidence to show that one ligand would be favored over the other.

Studies in humans with various forms of PTH have demonstrated an anabolic effect on bone, and have prompted significant interest in its use for the treatment of osteoporosis and related bone disorders. The significant anabolic effects of PTH on bone, including stimulation of bone formation which results in a net gain in bone mass and/or strength, have been demonstrated in many animal models and in humans.

It is commonly believed that PTH administration in humans and in relevant animal models has a negative effect on cortical bone. In fact, naturally occurring increases in endogenous PTH, which occur in the disorder hyperparathyroidism, result in thinning of cortical bone accompanied by an increase in connectivity and mass of trabecular bone. Past studies suggest that when Haversian cortical bone (found in humans and higher mammals) remodels under the influence of PTH, there will be a re-distribution of bone such that cortical bone mass and strength decrease, while trabecular bone increases in mass and strength. For example, in published clinical studies of administering PTH, cortical bone mass decreased after treatment with exogenous PTH and these findings have raised concern that treatment with PTH will lead to reduced cortical bone mass and strength. One concern raised by such studies is that there would be a loss of total skeletal bone mass due to the loss of cortical bone. This is of high clinical relevance as, in osteoporosis, the greater loss of trabecular bone compared to loss of cortical bone, means that mechanical loading is predominantly borne by the remaining cortical bone. Continued loss of cortical bone would increase the fracture risk. Therefore, it is important that a therapeutic agent for osteoporosis maintain or increase residual cortical bone.

The effects of PTH on cortical bone have been investigated in nonhuman animals with Haversian remodeling, such as dogs, ferrets, sheep and monkeys, but sample sizes are typically too small for reliable statistical analysis. The impact of the changes induced by PTH treatment on mechanical properties of cortical bone in such animals remains unknown. Published studies of rodents have shown increased cortical bone mass during administration of PTH but a loss of this benefit after withdrawal of PTH. However, rodent cortical bone has a distinctly different structure from Haversian cortical bone, and remodels by surface appositional formation and resorption, rather than by intracortical remodeling of osteons. Furthermore, technological limitations in biomechanical testing on the relatively short bones of rodents give rise to artifacts of measurement when an agent, such as a PTH, alters bone geometry to thicken the bone. Such artifacts make extrapolation of rat cortical bone responses to those of humans or other animals with osteonal remodeling unreliable. Therefore, the existing data for animals, like humans, undergoing Haversian remodeling indicates that PTH may have an adverse impact on cortical bone, causing net loss of bone mass through depletion of cortical bone.

As a consequence, it has been a popular belief regarding the action of PTH that patients may not achieve sufficient benefit from administration of PTH to justify its use. In fact, it is commonly believed that patients require additional drug therapy to treat or prevent conditions or disorders that accompany osteoporosis or bone trauma. For example it is believed that osteoporosis patients require concurrent or subsequent treatment with an antiresorptive to minimize loss of bone induced by PTH. It was also believed that patients would require additional medications to reduce the risk of or to treat disorders such as cancer, diabetes, cerebrovascular disorder, and other disorders that affect subjects that might otherwise benefit from administration of PTH. In fact, this model requiring additional therapeutic agents for additional indications has been the basis for several clinical studies in women. For example, three clinical studies have used PTH in post-menopausal women undergoing concurrent therapy with calcitonin or estrogen, or in premenopausal women taking GnRH agonist, Synarel, for endometriosis. The opposing effects of estrogen and PTH on cortical bone turnover make it particularly difficult to observe effects of just PTH during combination therapy with these two agents.

US Patent number 5,840,690 discloses the use of human parathyroid hormone or a fragment thereof (e.g. PTH(1-34)) in the treatment of cancer.

### SUMMARY OF THE INVENTION

The present invention includes the use, for the manufacture of a medicament for decreasing the risk of carcinoma in a subject, of a parathyroid hormone. The medicament preferably decreases the risk of skin carcinoma and breast carcinoma, preferably breast carcinoma. A preferred subject for the use of the invention is a human subject at risk of developing a carcinoma, particularly breast carcinoma, skin carcinoma, bladder carcinoma, gastric carcinoma, or a combination thereof. The subject at risk of developing carcinoma may have a relatively low risk of osteoporosis or a high risk of or be suffering from osteoporosis. In one preferred embodiment, the subject is a woman identified as having a relatively high risk of breast cancer. The-high risk of breast cancer may be based on known risk factors including age at menarche, age at first live birth, number of previous biopsies, and number of first-degree relatives with breast cancer. The high risk of breast cancer also may be based on a relatively high level of bioavailable serum estradiol or of free testosterone. In another preferred embodiment, the subject is a woman identified as having a high risk of or as suffering from osteoporosis, preferably a postmenopausal woman. A preferred subject in this embodiment is not concurrently taking hormone replacement therapy (HRT), estrogen or equivalent therapy, or antiresorptive therapy. In one embodiment, the patient also receives supplements of calcium and/or vitamin D.

A parathyroid hormone, such as the N-terminal amino acids 1-34 of recombinant human parathyroid hormone, can be administered either cyclically or intermittently. Preferably, this hormone is administered in a daily dose in the range of at least about 15 µg to about 40 µg, for at least about 12 months. The hormone is administered with or without concurrent administration of an antiresorptive agent, including vitamin D or calcium. In another embodiment, the invention provides an article of manufacture comprising packaging material and a pharmaceutical composition contained within that packaging material, where the composition comprises a parathyroid-hormone consisting of amino acid sequence 1-34 of human parathyroid and the packaging material comprising printed matter which indicates that the composition is effective for reducing the risk of carcinoma in human subject in need thereof when administered according to the present invention.

### DETAILED DESCRIPTION

The invention relates to the use of a parathyroid hormone in the manufacture of a medicament for reducing the risk of carcinoma in a human subject. In one embodiment the invention relates to reducing the risk of breast carcinoma and/or skin carcinoma, preferably breast carcinoma. In another embodiment, the invention relates to reducing the risk of carcinoma in a subject at risk for suffering from osteoporosis and/or bone fracture, by administering a parathyroid hormone.

As used herein, reducing risk or incidence includes decreasing the probability or incidence of an indication, disease, or disorder for a subject compared to a relevant, e.g. untreated, control population, or in the same subject prior to treatment according to the invention. Reduced risk or incidence can include delaying or preventing the onset of an indication, disease, or disorder. In some circumstances the occurrence of the disorder is reduced to the extent that the subject does not present any signs of the indication, disease, or disorder during and/or after the treatment period.

Additional aspects of methods employing administration of a parathyroid hormone are described in U.S. Patent Application No. 60/099,746 and PCT Patent Application No. PCT/US99/18961, published as WO 00/10596 on 2 March 2000, which claims priority to the above U.S. application.

### Cancer

The method of the invention benefits a subject at risk of developing carcinoma by decreasing the probability that the subject gets carcinoma. As used herein, the term "cancer" includes any cellular tumor, or mass, that, when not treated, grows. As used herein, the term "carcinoma" includes any cancer that arises from epithelial tissue. The method of the invention can reduce the risk of a variety of carcinomas, such as skin carcinoma, breast carcinoma, bladder carcinoma, gastric carcinoma, and the like.

If left untreated, a cancer typically invades other tissues, spreads, and eventually results in death. By reducing the incidence of cancer, the present invention prevents or reduces the likelihood of this invasion, spread, and death. Cancers can arise from a variety of causes, and the present invention can be effective in reducing risk of cancers due to, for example, increased age, family history of cancer, exposure to chemical carcinogens, an immunosuppressive drug, viral infection, or physical factors such as radiation. Many cancers are thought to involve genetic mutations that result in, for example, the conversion of protooncogenes to oncogenes and/or dysfunction of tumor suppressor genes. The method of the present invention can, for example, reduce the risk of breast cancer in subjects carrying mutations associated with breast cancer and retinoblastoma, such as mutations of the *BRCA1* gene or the *RBI* gene.

### Breast Carcinoma

The method of the invention can benefit a subject at risk of developing breast cancer by reducing the probability that they get carcinoma. The use of the invention can reduce the risk of various types of breast carcinoma, for example, those cancers due to or correlated with a genetic mutation in a tumor suppressor gene, e.g. *p53, BRCA1,* and the like. Risk of breast carcinoma of sporadic origin, or due to, for example, increased age, family-history of cancer, exposure to chemical carcinogens, an immunosuppressive drug, viral infection, or physical factors such as radiation can also be reduced by the method of the invention. The present use can reduce the risk of estrogen dependent and estrogen independent breast carcinoma. Preferably, the method of the invention is employed to reduce breast carcinoma in a woman.

More particularly, populations considered to be in need of treatment according to the present invention to reduce high risk of breast cancer can be defined using the Gail model. Gail MI-I Brinton LA, Byar DP et al (1989) Projecting individualized probabilities of developing breast cancer for white females who are being examined annually. J Natl Cancer Inst 81:1879-1886. This paper discloses a method to estimate the chance that a woman with given age and risk factors will develop breast cancer over a specified interval. The risk factors used were age at menarche, age at first live birth, number of previous biopsies, and number of first-degree relatives with breast cancer. A model of relative risks for various combinations of these factors was developed from case-control data from the Breast Cancer Detection Demonstration Project (BCDDP). The model allowed for the fact that relative risks associated with previous breast biopsies were smaller for women aged 50 or more than for younger women. Thus, the proportional hazards models for those under age 50 and for those of age 50 or more. The baseline age-specific hazard rate, which is the rate for a patient without identified risk factors, is computed as the product of the observed age-specific composite hazard rate times the quantity 1 minus the attributable risk. The authors calculated individualized breast cancer probabilities from information on relative risks and the baseline hazard rate. The authors presented tables for estimating individualized absolute risks of developing breast cancer. They also pointed out the methods disclosed in this article may be used to help design prevention trials in high-risk populations, because an important determinant of the required sample size is the absolute risk of developing breast cancer in such a population.

Estrogen is thought to play a role in the etiology of certain breast cancers. For instance, see Cauley JA Lucas FL, Kuller LH, Stone K, Browner W, Cummings SR (1999). In particular, elevated serum estradiol and testosterone concentrations are associated with a high risk of breast cancer. Study of Osteoporotic Fractures Research Group. Annals of Inter Med. 130:270-277. This article discloses that measurement of sex hormone levels may identify women at high risk for breast cancer who should consider preventive therapies. In a prospective case-cohort study of 97 women with confirmed incident breast cancer and 244 randomly selected controls; sex-steroid hormone concentrations were assayed by using serum that was collected at baseline and stored at -190 degrees C. Risk factors for breast cancer were ascertained by questionnaire. Incident cases of breast cancer were confirmed by review of medical records during an average period of 3.2 years. The authors found that the relative risk for breast cancer in women with the highest concentration of bioavailable estradiol (≥6.83 pmol/L or 1.9 pg/mL) was 3.6 (95% CI, 1.3 to 10.0) compared with women with the lowest concentration. The risk for breast cancer in women with the highest concentration of free testosterone compared with those with the lowest concentration was 3.3 (CI, 1.1 to 10.3). The estimated incidence of breast cancer per 1000 person-years was 0.4 (CI, 0.0 to 1.3) in women with the lowest levels ofbioavailable estradiol and free testosterone compared with 6.5 (CI, 2.7 to 10.3) in women with the highest concentrations of these hormones. Traditional risk factors for breast cancer were similar in case-patients and controls. Adjustments for these risk factors had little effect on the results. The authors therefore concluded that estradiol and testosterone levels may play important roles in the development of breast cancer in older women. Further, a single measurement ofbioavailable estradiol and free testosterone may be used to estimate a woman's risk for breast cancer. The authors also noted that women identified as being at high risk for breast cancer as determined by these hormone levels may benefit from antiestrogen treatment for primary prevention. According to the present invention, women identified as being at high risk for breast cancer as determined by these same hormone levels also would benefit from treatment with parathyroid hormone as taught herein.

Subjects who would benefit from reduction of risk for developing cancer by the present invention may or may not also be at relatively high risk of or suffering from osteoporosis or osteopenia. For instance, while elevated serum estradiol concentrations are associated with a high risk of breast cancer, low levels of serum estradiol concentrations are associated with low bone density and increased risk of fractures among elderly women with osteoporosis. See Ettinger B, Pressman A, Sklarin P, Bauer DC, Cauley JA, Cummings SR (1998) Associations between low levels of serum estradiol, bone density and fractures among elderly women: the study of osteoporotic fractures. J Clin Endocrinol Metab 83:2239-2243. More particularly, this report discloses a study to evaluate the skeletal effects of endogenous serum estradiol. The authors measured bone mineral density (BMD) at the calcaneus and radius (single photon absorptiometry) and at the hip and spine (dual x-ray absorptiometry) in 274 women aged 65 yr or more who participated in the Study of Osteoporotic Fractures. Lateral radiographs of the thoracic and lumbar spine were also taken, and serum was assayed for estradiol. Those who had estradiol levels from 10-25 pg/mL had 4.9%, 9.6%, 7.3%, and 6.8% greater BMD at total hip, calcaneus, proximal radius, and spine than those with levels below 5 pg/mL. After multiple adjustments, BMD differences remained statistically significant and corresponded to about 0.4 SD. Vertebral deformities were less prevalent among women whose estradiol level exceeded 5 pg/mL; the multiple adjusted odds ratio was 0.4 (95% confidence interval, 0.2-0.8). The authors concluded that physiologically low estradiol has a salutary effect on the skeleton in elderly women, possibly by reducing skeletal remodeling. Accordingly, women identified as being at highest risk for breast cancer as determined by serum estradiol levels, as discussed above, would be expected to be at lowest risk for developing osteoporosis.

The present invention also reduces the need to administer estrogen to a population at risk of estrogen-dependent breast cancer. The method of the present invention provides a method that does not require estrogen replacement therapy, that provides reduction in the risk of breast cancer, and that also reduces the effects or progression of osteoporosis and/or the reduces risks of osteoporosis and bone trauma.

### Skin Carcinoma

The use of the invention can benefit a subject at risk of developing skin carcinoma by reducing the probability that they get carcinoma. The use of the invention can reduce the risk of skin carcinoma due to a genetic mutations and/or exposure to radiation, such as sunlight in the form of ultraviolet radiation. The present use also can reduce risk of skin carcinoma due to, for example, increased age, family history of cancer, exposure to chemical carcinogens, an immunosuppressive drug, viral infection, and the like.

### Bone Trauma

A human can suffer any of a variety of bone traumas due, for example, to accident, medical intervention, disease, or disorder. In the young, bone trauma is likely due to fracture, medical intervention to repair a fracture, genetic disorders that increase susceptibility to bone fracture or bone lytic lesions, or the repair of joints or connective tissue damaged, for example, through athletics. Other types of bone trauma, such as those from osteoporosis, degenerative bone disease (such as arthritis or osteoarthritis), hip replacement, or secondary conditions associated with therapy for other systemic conditions (e.g., glucocorticoid osteoporosis, bums or organ transplantation) are found most often in older people.

Bone trauma can be a problem for subjects at risk of cancer. For example, many subjects with the disorders described above also are at risk of or have some risk factors for cancer, such as skin cancer or breast cancer. In particular, women with or at risk of osteoporosis can also be at risk of breast carcinoma. The method of the invention can benefit these types of subjects.

Preferred subjects are women, at risk for or suffering from osteoporosis. Risk factors for osteoporosis are known in the art and include hypogonadal conditions in men and women, irrespective of age, conditions, diseases or drugs that induce hypogonadism, nutritional factors associated with osteoporosis (low calcium or vitamin D being the most common), smoking, alcohol, drugs associated with bone loss (such as glucocorticoids, thyroxine, heparin, lithium, anticonvulsants etc.), loss of eyesight or walking styles that predisposes to falls, space travel, immobilization, chronic hospitalization or bed rest, and other systemic diseases that have been linked to increased risk of osteoporosis. Indications of the presence of osteoporosis are known in the art and include radiological evidence of at least one vertebral compression fracture, low bone mass (typically at least 1 standard deviation below mean young normal values), and/or atraumatic fractures.

The use of the invention can benefit subjects suffering from, or at risk of, osteoporosis by reducing risk of carcinoma, such as breast carcinoma. The present invention provides a use, in particular, effective for reducing risk of carcinoma, such as breast carcinoma, in a subject with or at risk of progressing to osteoporosis or patients in which spinal osteoporosis may be progressing rapidly. A typical woman at risk for osteoporosis is a postmenopausal woman or a premenopausal, hypogonadal woman. A preferred subject is a postmenopausal woman who is not concurrently taking hormone replacement therapy (HRT), estrogen or equivalent therapy, or antiresorptive therapy. The use of invention can benefit a subject at any stage of osteoporosis, but especially in the early and advanced stages.

### Parathyroid Hormone

As active ingredient, the composition or solution may incorporate the full length, 84 amino acid form of parathyroid hormone, particularly the human form, hPTH (1-84), obtained either recombinantly, by peptide synthesis or by extraction from human fluid. See, for example, U.S. Pat. No. 5,208,041. The amino acid sequence for hPTH (1-84) is reported by Kimura et al. in Biochem. Biophys. Res. Comm., 114(2):493.

The composition or solution may also incorporate as active ingredient fragments or variants of fragments of human PTH or of rat, porcine or bovine PTH that have human PTH activity as determined in the ovariectomnized rat model of osteoporosis reported by Kimmel et al., Endocrinology, 1993, 32(4):1577.

The parathyroid hormone fragments desirably incorporate at least the first 28 N-terminal residues, such as PTH(1-28), PTH(1-31), PTH(1-34), PTH(1-37), PTH(1-38) and PTH(1-41). Alternatives in the form of PTH variants incorporate from 1 to 5 amino acid substitutions that improve PTH stability and half-life, such as the replacement of methionine residues at positions 8 and/or 18 with leucine or other hydrophobic amino acid that improves PTH stability against oxidation and the replacement of amino acids in the 25-27 region with trypsin-insensitive amino acids such as histidine or other amino acid that improves PTH stability against protease. Other suitable forms of PTH include PTHrP, PTHrP(1-34), PTHrP(1-36) and analogs of PTH or PTHrP that activate the PTH1 receptor. These forms of PTH are embraced by the term "parathyroid hormone" as used generically herein. The hormones may be obtained by known recombinant or synthetic methods, such as described in U.S. Pat. Nos. 4,086,196 and 5,556,940.

The preferred hormone is human PTH(1-34). Stabilized solutions of human PTH(1-34), such as recombinant human PTH(1-34) (rhPTH(1-34)), that can be employed in the present method are described in U.S. Patent Application Serial No. 60/069,075. Crystalline forms of human PTH(1-34) that can be employed in the present method are described in U.S. Patent Application Serial No. 60/069,875.

### Administering Parathyroid Hormone

A parathyroid hormone can typically be administered parenterally, preferably by subcutaneous injection, by methods and in formulations well known in the art. Stabilized formulations of human PTH(1-34) that can advantageously be employed in the present method are described in U.S. Patent Application Serial No.60/069,075. This patent application also describes numerous other formulations for storage and administration of parathyroid hormone. A stabilized solution of a parathyroid hormone can include a stabilizing agent, a buffering agent, a preservative, and the like.

The stabilizing agent incorporated into the solution or composition includes a polyol which includes a saccharide, preferably a monosaccharide or disaccharide, e.g., glucose, trehalose, raffinose, or sucrose; a sugar alcohol such as, for example, mannitol, sorbitol or inositol, and a polyhydric alcohol such as glycerine or propylene glycol or mixtures thereof. A preferred polyol is mannitol or propylene glycol. The concentration of polyol may range from about 1 to about 20 wt-%, preferably about 3 to 10 wt-% of the total solution.

The buffering agent employed in the solution or composition of the present invention may be any acid or salt combination which is pharmaceutically acceptable and capable of maintaining the aqueous solution at a pH range of 3 to 7, preferably 3-6. Useful buffering systems are, for example, acetate, tartrate or citrate sources. Preferred buffer systems are acetate or tartrate sources, most preferred is an acetate source. The concentration of buffer may be in the range of about 2 mM to about 500 mM, preferably about 2 mM to 100 mM.

The stabilized solution or composition of the present invention may also include a parenterally acceptable preservative. Such preservatives include, for example, cresols, benzyl alcohol, phenol, benzalkonium chloride, benzethonium chloride, chlorobutanol, phenylethyl alcohol, methyl paraben, propyl paraben, thimerosal and phenylmercuric nitrate and acetate. A preferred preservative is m-cresol or benzyl alcohol; most preferred is m-cresol. The amount of preservative employed may range from about 0.1 to about 2 wt-%, preferably about 0.3 to about 1.0 wt-% of the total solution.

Thus, the stabilized PTH solution can contain mannitol, acetate and m-cresol with a predicted shelf-life of over 15 months at 5°C.

The parathyroid hormone compositions can, if desired, be provided in a powder form containing not more than 2% water by weight, that results from the freeze-drying of a sterile, aqueous hormone solution prepared by mixing the selected parathyroid hormone, a buffering agent and a stabilizing agent as above described. Especially useful as a buffering agent when preparing lyophilized powders is a tartrate source. Particularly useful stabilizing agents include glycine, sucrose, trehalose and raffinose.

In addition, parathyroid hormone can be formulated with typical buffers and excipients employed in the art to stabilize and solubilize proteins for parenteral administration. Art recognized pharmaceutical carriers and their formulations are described in Martin, "Remington's Pharmaceutical Sciences," 15th Ed.; Mack Publishing Co., Easton (1975). A parathyroid hormone can also be delivered via the lungs, mouth, nose, by suppository, or by oral formulations.

The parathyroid hormone is formulated for administering a dose effective for reducing a subject's risk of carcinoma, particularly breast carcinoma. Preferably, a subject receiving parathyroid hormone also receives effective doses of calcium and vitamin D, which can enhance the effects of the hormone. An effective dose of parathyroid hormone is typically greater than about 5 µg/kg/day although, particularly in humans, it can be as large at about 10 to about 40 µg/day per subject, regardless of body mass, or larger as is effective for reducing a subject's risk of carcinoma, particularly breast carcinoma. A subject suffering from hypoparathyroidism can require additional or higher doses of a parathyroid hormone; such a subject also requires replacement therapy with the hormone. Doses required for replacement therapy in hypoparathyroidism are known in the art. In certain instances, relevant effects of PTH can be observed at doses less than about 5 µg/kg/day, or even less than about 1 µg/kg/day.

The hormone can be administered regularly (e.g., once or more each day or week), intermittently (e.g., irregularly during a day or week), or cyclically (e.g., regularly for a period of days or weeks followed by a period without administration). Preferably PTH is administered once daily for 1-7 days per week over a period ranging from 3 months for up to 3 years in osteoporotic patients. Preferably, cyclic administration includes administering a parathyroid hormone for at least 2 remodeling cycles and withdrawing parathyroid hormone for at least 1 remodeling cycle. Another preferred regime of cyclic administration includes administering the parathyroid hormone for at least about 12 to about 24 months and withdrawing parathyroid hormone for at least 6 months. Typically, the benefits of administration of a parathyroid hormone persist after a period of administration. The benefits of several months of administration can persist for as much as a year or two, or more, without additional administration.

For hPTH(1-34) in particular, in studies by the present applicant the lowest tested dose found to be biologically active in human subjects, as indicated by detectable changes in various biochemical bone markers, was about 15µg; 6µg was found to produce no significant effects. Therefore, for reducing risk of carcinoma in men or women with hPTH(1-34) according to the present invention, preferably one should use a daily dose greater than about 6µg, more preferably at least about 15 µg. Daily doses of hPTH(1-34) of 20µg and 40µg were both found to be similarly effective against osteoporosis in both men and women and both also have been shown to reduce the risk of carcinoma in human subjects as shown, for instance, in Example 1, below. Higher daily doses of hPTH(1-34) have been used in human subjects previously, although it is believed that parathyroid hormone has never been shown to reduce the risk of carcinoma in human subjects. Therefore, any daily dose of hPTH(1-34) in the range of greater than about 6µg to at least about 40 µg would be effective for reduction of the risk of carcinoma, according to the present method of using this form of parathyroid hormone. However, this applicant has found that a daily dose of about 20µg produced fewer undesirable side effects in human subjects than a daily dose of about 40µg. Hence, daily doses above about 40µg are less preferred than doses of 40µg or less; and a daily dose of about 20µg is more preferred than any higher dose from this perspective. Accordingly, the present invention provides the use of a parathyroid hormone in the manufacture of a medicament for reducing the risk of carcinoma in a human subject at risk thereof. Preferably, the parathyroid hormone consists of amino acid sequence 1-34 of human parathyroid hormone. This hormone may be administered with or without concurrent administration of an antiresorptive agent other than vitamin D or calcium. Preferably, the hormone is administered in a daily dose in the range of about 15 µg to about 40 µg, for at least about 12 months. The hormone may be administered for longer periods of several years (e.g., up to 3 years as in osteoporotic patients), including for the remaining life of a human subject identified as having a high risk of carcinoma.

### Uses of Formulations of a Parathyroid Hormone

A kit including the present pharmaceutical compositions may be used with the methods of the present invention. The kit can contain a vial or other container, such as a pen-style injection device or a cartridge for such a device, which contains a formulation of the present invention and suitable carriers, either dried or in liquid form. The kit may further include instructions in the form of a label on the vial and/or in the form of an insert included in a box in which the vial or other container is packaged, for the use and administration of the compounds. The instructions can also be printed on the box in which the vial or container is packaged. The instructions contain information such as sufficient dosage and administration information so as to allow a worker in the field to administer the drug. It is anticipated that a worker in the field encompasses any doctor, nurse, or technician who might administer the drug or oversee self-administration of the drug by a human subject.

A pharmaceutical composition including a formulation of one or more parathyroid hormones, such as human PTH(1-84) or human PTH(1-34), which is suitable for parenteral administration, may used in the methods of the present invention. A formulation of one or more parathyroid hormones, such as human PTH(1-84) or human PTH(1-34), can be used for manufacturing a composition or medicament suitable for administration by parenteral administration. A liquid or solid formulation of one or more parathyroid hormones, such as human PTH(1-84) or human PTH(1-34), in a form that is suitable for parenteral administration, can be manufactured in several ways, using conventional techniques. A liquid formulation can be manufactured by dissolving the one or more parathyroid hormones, such as human PTH(1-84) or human PTH (1-34), in a suitable solvent, such as water, at an appropriate pH, including buffers or other excipients, for example, to form one of the stabilized solutions described hereinabove.

The example which follows is illustrative of the invention and is not intended to be limiting.

### EXAMPLE

### Example 1 - - Reduced Risk of Cancer Upon Administration of rhPTH(1-34) to Humans

- Number of Subjects:: rhPTH(1-34): 1093 enrolled, 848 finished. Placebo: 544 enrolled, 447 finished.
- Diagnosis and Inclusion Criteria:: Women ages 30 to 85 years, postmenopausal for a minimum of 5 years, with a minimum of one moderate or two mild atraumatic vertebral fractures.
- Dosage and Administration:: Test Product (blinded)
rhPTH(1-34): 20 µg/day, given subcutaneously rhPTH(1-34): 40 µg/day, given subcutaneously
Reference Therapy (blinded)
Placebo study material for injection
- Duration of Treatment:: rhPTH(1-34): 17-23 months (excluding 6-month run-in phase)
Placebo: 17-23 months (excluding 6-month run-in phase)
- Criteria for Evaluation:: Spine x-ray; serum biological markers (calcium, bone-specific alkaline phosphatase, procollagen I carboxy-terminal propeptide); urine markers (calcium, N-telopeptide, free deoxypyridinoline); 1,25-dihydroxyvitamin D; bone mineral density: spine, hip, wrist, and total body; height; population pharmacokinetics; bone biopsy (selected study sites).

| **Patient Characteristics** | | | | |
|---|---|---|---|---|
| | Placebo (N=544) | PTH-20 (N=541) | PTH-40 (N=552) | p-value |
| Caucasian | 98.9% | 98.9% | 98.4% | 0.672 |
| Age | 69.0±7.0 | 69.5±7.1 | 69.9±6.8 | 0.099 |
| Years post menopausal | 20.9±8.5 | 21.5±8.7 | 21.8±8.2 | 0.273 |
| Hysterectomized | 23.8% | 23.1% | 21.6% | 0.682 |
| Uterus+0 or 1 ovary | 57 | 51 | 58 | |
| Uterus + 2 ovaries | 61 | 57 | 51 | |
| Unknown | 11 | 17 | 10 | |
| Previous osteoporosis | 14.9% | 15.5% | 13.0% | 0.479 |
| drug use | | | | |
| Baseline spine BMD | 0.82±0.17 | 0.82±0.17 | 0.82±0.17 | >0.990 |
| Baseline # of vert. fx | | | | >0.990 |
| 0 | 54(10.4%) | 45(8.8%) | 54(10.1%) | |
| I | 144(27.8%) | 159(31.1%) | 169(31.6%) | |
| 2 | 128(24.7%) | 128(25.0%) | 125(23.4%) | |
| 3 | 75(14.5%) | 67 (13.1%) | 81 (15.1%) | |
| 4 | 59(11.4%) | 49(9.6%) | 45(8.4%) | |
| 5 | 28(5.4%) | 31 (6.1%) | 21(3.9%) | |
| 6 | 13(2.5%) | 20(3.9%) | 25(4.7%) | |
| 7 | 6(1.2%) | 7(1.4%) | 10(1.9%) | |
| 8 | 9(1.7%) | 5(1.0%) | 3(0.6%) | |
| 9 | 1(0.2%) | 0 | 2 (0.4%) | |
| 10 | 1(0.2%) | 1(0.2%) | 0 | |
| Unspecified | 26 | 29 | 17 | |

### Results

Table 1 illustrates data showing the reduction in the number of patients having detectable tumors upon treatment with PTH.

**Table 1.**

| Effect of PTH on the incidence of cancer expressed as number (and percentage) of patients with detectable tumors | | | | |
|---|---|---|---|---|
| | Placebo (N=544) | PTH-20 (N=541) | PTH-40 (N=522) | p-value |
| Breast carcinoma | 7 (1.29%) | 1 (0.185%) | 1 (0.192%) | 0.017 |
| Skin carcinoma | 5 (0.919%) | 2 (0.370%) | 4 (0.766%) | 0.532 |
| All cancers | 21 (3.86%) | 8 (1.48%) | 11(2.11%) | 0.03 |
| All cancers except breast carcinoma | 16 (2.94%) | 7 (1.30%) | 10 (1.92%) | |

The percentage of patients with any form of detectable cancer was significantly lower in PTH-treated patients than in placebo-treated patients (1.79% vs. 3.86%, respectively, p=0.03) when all PTH-treated patients were compared to the placebo control group. The percentage of PTH patients with detectable cancer at 20 µg/day was 1.48% and at 40 µg/day was 2.11 % (Table 1).

Similarly, the percentage of patients with breast carcinoma was significantly lower in PTH-treated patients than in placebo-treated patients (0.188% vs. 1.29%, respectively, p=0. 017) when all PTH-treated patients were compared to the placebo-treated group. The percentage of PTH patients with detectable breast carcinoma at 20 µg/day was 0.185% and at 40 µg/day was 0.195% (Table 1). Although subjects included in this study had 1 or more fractures, many had bone mass within an age-matched normal range. Thus, subjects in this clinical trial might be expected to show a comparable incidence of breast cancer to that of the general population.

The percentage of patients with skin carcinoma was also lower in PTH-treated patients than in placebo-treated patients (0.564% vs. 0.919%, respectively, p=0.532). The percentage of PTH patients with detectable skin carcinoma at 20 µg/day was 0.370% and at 40 µg/day was 0.766% (Table 1).

Additionally, the percentage of patients with any form of detectable cancer, excluding breast carcinoma, was lower in PTH-treated patients than in placebo-treated patients (1.60% vs. 2.94%, respectively) when all PTH-treated patients were compared to placebo. The percentage of PTH patients with detectable cancer, excluding breast carcinoma, at 20µg/day was 1.30% and at 40 µg/day was 1.92% (Table 1).

In summary, the data presented above indicate that patients treated with PTH have a reduced risk of cancer. Particularly, the incidence of breast cancer was significantly lower in the PTH-treated patient population relative to placebo controls. Furthermore, the total incidence of any form of cancer was significantly lower in PTH-treated patients relative to placebo controls. This decrease in the incidence of cancer in PTH-treated patients is not entirely due to the reduction in the incidence of breast carcinoma, as the trend towards lower incidence of cancer remains when breast cancer is excluded. Therefore, administration of PTH decreases the risk of cancer.

### Discussion

These data on cancer are the first data on the reduction of cancer risk by PTH in humans. These findings demonstrate a significantly lower incidence of overall cancer. In addition, certain cancers, skin carcinoma and breast carcinoma, were reduced in patients administered PTH. Of particular interest is the lower incidence of breast cancer observed in patients treated with PTH relative to placebo controls. Investigation of the records of affected patients indicated that this reduction in incidence of new breast cancer cases was a treatment-dependent phenomenon.

The invention has been described with reference to various specific and preferred embodiments and techniques. However, it should be understood that many variations and modifications may be made while remaining within the scope of the invention. All publications and patent applications in this specification are indicative of the level of ordinary skill in the art to which this invention pertains.

## Claims

1. Use of a parathyroid hormone in the manufacture of a medicament for reducing risk of carcinoma in a human subject.

2. The use as claimed in claim 1, wherein the medicament reduces the probability of the human subject getting a carcinoma.

3. The use of claim 1 or claim 2, wherein the carcinoma comprises breast carcinoma, skin carcinoma, bladder carcinoma, gastric carcinoma, or a combination thereof.

4. The use of claim 3, wherein the carcinoma comprises breast carcinoma.

5. The use of claim 4, wherein the breast carcinoma comprises estrogen dependent carcinoma.

6. The use of claim 4, wherein the breast carcinoma comprises estrogen independent carcinoma.

7. The use of any one of the preceding claims, wherein the subject is a woman identified as having a relatively high risk of breast cancer.

8. The use of claim 7 wherein the high risk of breast cancer is based on age at menarche, age at first live birth, number of previous biopsies, and number of first-degree relatives with breast cancer.

9. The use of claim 7 wherein the high risk of breast cancer is based a relatively high level ofbioavailable serum estradiol or of free testosterone.

10. The use of any one of the preceding claims, wherein the subject is a woman identified as having a high risk of or as suffering from osteoporosis.

11. The use of claim 10, wherein the subject is a postmenopausal woman.

12. The use of claim 11, wherein the woman is not concurrently taking hormone replacement therapy or an antiresorptive.

13. The use of any one of claims 10 to 12, wherein the subject is a woman in an early stage of osteoporosis or in an advanced stage of osteoporosis.

14. The use of any one of the preceding claims, wherein the parathyroid hormone comprises a fragment of a parathyroid hormone selected from the group consisting of PTH (1-31), PTH (1-34), PTH (1-37), PTH (1-38), and PTH (1-41).

15. The use of claim 14, wherein the parathyroid hormone is human PTH (1-34).

16. The use of any one of claims 1 to 13, wherein the parathyroid hormone is human PTH (1-84).

17. The use of any one of claims 1 to 15 wherein the medicament comprises parathyroid hormone consisting of amino acid sequence 1-34 of human parathyroid hormone for administering to a subject in a daily dose of at least 15 µg to about 40 µg for at least 12 months.

18. The use of any one of the preceding claims wherein the medicament is for use with the administration of calcium, vitamin D, or a combination thereof.

19. The use of any one of the preceding claims wherein the medicament is suitable for parenteral administration.

## Patentansprüche

1. Verwendung eines Parathormons zur Herstellung eines Arzneimittels zur Verringerung des Carzinomrisikos bei einem Menschen.

2. Verwendung nach Anspruch 1, worin das Arzneimittel die Wahrscheinlichkeit eines Menschen verringert, ein Carzinom zu bekommen.

3. Verwendung nach Anspruch 1 oder 2, worin das Carzinom Brustcarzinom, Hautcarzinom, Blasencarzinom, Magencarzinom oder eine Kombination hiervon umfasst.

4. Verwendung nach Anspruch 3, worin das Carzinom Brustcarzinom umfasst.

5. Verwendung nach Anspruch 4, worin das Brustcarzinom ein Östrogen-abhängiges Carzinom umfasst.

6. Verwendung nach Anspruch 4, worin das Brustcarzinom ein Östrogen-abhängiges Carzinom umfasst.

7. Verwendung nach einem der vorangehenden Ansprüche, worin der Mensch eine Frau ist, bei der ein relativ hohes Risiko für Brustkrebs identifiziert wurde.

8. Verwendung nach Anspruch 7, worin das hohe Risiko für Brustkrebs auf dem Lebensalter bei der Menarche, dem Lebensalter bei der Erstgeburt, der Anzahl an vorangehenden Biopsien und der Anzahl an Verwandten ersten Grades mit Brustkrebs basiert.

9. Verwendung nach Anspruch 7, worin das hohe Risiko für Brustkrebs auf ein hohes Niveau an bioverfügbarem Serumöstradiol oder freiem Testosteron basiert.

10. Verwendung nach einem der vorangehenden Ansprüche, worin der Mensch eine Frau ist, bei der ein hohes Risiko für Osteoporose identifiziert wurde oder die daran leidet.

11. Verwendung nach Anspruch 10, worin der Mensch eine postmenopausale Frau ist.

12. Verwendung nach Anspruch 11, worin die Frau nicht gleichzeitig eine Hormonersatztherapie oder ein Antiresorptivum einnimmt.

13. Verwendung nach einem der Ansprüche 10 bis 12, worin der Mensch eine Frau in einem frühen Stadium von Osteoporose oder einem fortgeschrittenen Stadium von Osteoporose ist.

14. Verwendung nach einem der vorangehenden Ansprüche, worin das Parathormon ein Fragment eines Parathormons umfasst, das aus der Gruppe ausgewählt, die besteht aus PTH (1-31), PTH (1-34), PTH (1-37), PTH (1-38) und PTH (1-41).

15. Verwendung nach Anspruch 14, worin das Parathormon humanes PTH (1-34) ist.

16. Verwendung nach einem der Ansprüche 1 bis 13, worin das Parathormon humanes PTH (1-84) ist.

17. Verwendung nach einem der Ansprüche 1 bis 15, worin das Arzneimittel Parathormon umfasst, das aus der Aminosäuresequenz 1-34 des humanen Parathormons besteht, zur Verabreichung an einen Menschen in einer Tagesdosis von mindestens 15 µg bis etwa 40 µg für mindestens 12 Monate.

18. Verwendung nach einem der vorangehenden Ansprüche, worin das Arzneimittel zur Verwendung mit der Verabreichung von Calcium, Vitamin D oder einer Kombination hiervon vorgesehen ist.

19. Verwendung nach einem der vorangehenden Ansprüche, worin das Arzneimittel zur parenteralen Verabreichung geeignet ist.

## Revendications

1. Utilisation d'une parathormone dans la fabrication d'un médicament pour réduire le risque de carcinome chez un sujet humain.

2. Utilisation selon la revendication 1, dans laquelle le médicament réduit la probabilité pour le sujet humain de contracter un carcinome.

3. Utilisation selon la revendication 1 ou la revendication 2, dans laquelle le carcinome comprend un carcinome du sein, un carcinome de la peau, un carcinome de la vessie, un carcinome gastrique, ou une combinaison de ceux-ci.

4. Utilisation selon la revendication 3, dans laquelle le carcinome comprend un carcinome du sein.

5. Utilisation selon la revendication 4, dans laquelle le carcinome du sein comprend un carcinome oestrogéno dépendant.

6. Utilisation selon la revendication 4, dans laquelle le carcinome du sein comprend un carcinome oestrogéno non dépendant.

7. Utilisation selon l'une quelconque des revendications précédentes, dans laquelle le sujet est une femme que l'on identifie comme ayant un risque relativement élevé de cancer du sein.

8. Utilisation selon la revendication 7, dans laquelle on base ledit risque élevé de cancer du sein sur l'âge des premières règles, sur l'âge du premier accouchement, sur le nombre de biopsies précédentes, et sur le nombre de parents au premier degré ayant un cancer du sein.

9. Utilisation selon la revendication 7, dans laquelle on base ledit risque élevé de cancer du sein sur un niveau relativement élevé d'oestradiol sérique bio disponible ou de testostérone libre.

10. Utilisation selon l'une quelconque des revendications précédentes, dans laquelle le sujet est une femme que l'on identifie comme ayant un risque élevé ou souffrant d'ostéoporose.

11. Utilisation selon la revendication 10, dans laquelle le sujet est une femme post ménopausée.

12. Utilisation selon la revendication 11, dans laquelle la femme ne prend pas en même temps un traitement de remplacement hormonal ni un agent anti résorption.

13. Utilisation selon l'une quelconque des revendications 10 à 12, dans laquelle le sujet est une femme dans un stade précoce d'ostéoporose ou dans un stade d'ostéoporose avancé.

14. Utilisation selon l'une quelconque des revendications précédentes, dans laquelle la parathormone comprend un fragment d'une parathormone que l'on choisit parmi le groupe constitué de la PTH(1 à 31), de la PTH(1 à 34), de la PTH(1 à 37), de la PTH(1 à 38), et de la PTH(1 à 41).

15. Utilisation selon la revendication 14, dans laquelle la parathormone est la PTH(1 à 34) humaine.

16. Utilisation selon l'une quelconque des revendications 1 à 13, dans laquelle la parathormone est la PTH(1 à 84) humaine.

17. Utilisation selon l'une quelconque des revendications 1 à 15, dans laquelle le médicament comprend une parathormone composée d'une séquence d'acides aminés 1 à 34 de parathormone humaine pour une administration à un sujet à une dose journalière d'au moins 15 µg à environ 40 µg pendant au moins 12 mois.

18. Utilisation selon l'une quelconque des revendications précédentes dans laquelle le médicament est pour une utilisation avec l'administration de calcium, de vitamine D, ou d'une combinaison de ceux-ci.

19. Utilisation selon l'une quelconque des revendications précédentes, dans laquelle le médicament est approprié pour une administration parentérale.
